# EUROPEAN PATENT SPECIFICATION

(11) **EP 1 706 081 B1**
(45) Date of publication and mention of the grant of the patent: **06.05.2009**
(21) Application number: 04812818.5
(22) Date of filing: 03.12.2004
(51) Int. Cl.: A61F 9/08, G01J 3/50, G06T 7/40

(54) **SYSTEM AND METHOD FOR IDENTIFYING AT LEAST ONE COLOR FOR A USER**
SYSTEM UND VERFAHREN ZUR IDENTIFIZIERUNG MINDESTENS EINER FARBE FÜR EINEN ANWENDER
SYSTEME ET PROCEDE DESTINES A L'IDENTIFICATION D'AU MOINS UNE COULEUR POUR UN UTILISATEUR

(30) Priority: 03.12.2003 US 526782 P
(43) Date of publication of application: 04.10.2006
(73) Proprietor: TENEBRAEX CORPORATION, Boston, Massachusetts 02210 (US)
(72) Inventor: JONES, Peter, W., J., Belmont, MA 02478 (US)
(74) Representative: Wilson, Alan Stuart
(86) International application number: PCT/US2004/040380
(87) International publication number: WO 2005/055893

(56) References cited:
- DE-A1- 4 400 021
- DE-A1- 10 157 921
- DE-A1- 19 838 806
- FR-A- 2 811 425

## Description

### CROSS-REFERENCE TO RELATED APPLICATIONS

This application claims priority to and benefit of U.S. Provisional Patent Application No. 60/526,782, filed on 3 December 2003. This application also claims priority to and benefit of U.S. Patent Application 10/388,803, filed on 13 March 2003.

### BACKGROUND

Color vision impairment is a condition that affects a significant portion of the population. Approximately one of every twenty five people suffers from red-green color-vision impairment. Six to eight percent of the male population is red-green color-vision impaired. A red-green color-vision impaired observer is generally unable to distinguish between green and red, as well as yellow and any of the shades of orange, which are formed from combinations of red and green.

For these people visual discrimination of color-coded data is difficult, if not practically impossible, when green, red, or yellow data are adjacent in a scene or image. In the color space of such persons, the red-green hue dimension is missing, and red and green are both seen as yellow; they have primarily a yellow-blue dimension.

Even people with normal color vision can, at times, have difficulty distinguishing between colors. Lenses of the eyes tend to cloud with aging, due to a host of causes, such as cataracts. The elderly often experience changes in their ability to sense colors, and many see objects as if they have been viewed through yellowish filters. Additionally, over time, ultraviolet rays degenerate proteins in the eyes, and light having short wavelengths is absorbed and blue-cone sensitivity is thereby reduced. As a result, the appearance of most, if not all, colors changes, yellow tending to predominate, or a blue or a bluish violet color tending to become darker. Specifically, "white and yellow," "blue and black," and "green and blue" gradually become more difficult to distinguish. Similarly, even a healthy individual with "normal" vision can perceive colors differently when he or she is at an altitude greater than what he or she is normally used to, or when under certain medications.

Software programs assisting color-vision impaired or other observers distinguish between colors do exist, but have been limited primarily to configuring computers so that the observer can move a pointer over various positions on the computer's display monitor and be cued with information indicative of color content of the object pointed to by the pointer. However, such prior art systems and methods, although helpful, have utility only for images viewed on a computer and fail to provide solutions for most activities of daily living.

DE 19838806 discloses a device which aids weak-sighted or blind people. The device of this document relates to methods of optical object detection and falls under the definition of the preamble of claim 8.

### SUMMARY OF THE INVENTION

There is therefore a need for systems and methods to identify one or more colors for a user, for example a color-vision impaired observer, while at the same time enabling the user to choose, in real time or otherwise, an image of a scene of interest, from which the colors are identified. In one aspect, the systems and methods described herein integrate with a commercial portable electronic device to allow the user to capture an image of a scene of interest; display the captured image on a display screen associated with the portable device; and identify for the user one or more colors of one or more positions or regions, selected by the user, in the image, and to do so in a form and manner perceptible to the user.

The method and system of the invention are defined in the appended claims.

In one embodiment, the systems and methods described herein operatively cooperate or integrate with a commercial cellular telephone, equipped with a digital camera, that would allow a color-vision impaired or other user to differentiate colors in an image captured by the digital camera. Once the user has taken a picture of a scene have an object or group of objects, the software program, in one embodiment, provides the user with a visual or auditive cue indicative of the color of the object that a cursor, movable by the user, is over at any given time, thus allowing the user to distinguish between colors in the image.

In another embodiment, the systems and methods described herein can be used on real-time images that the camera device captures as the user aims the camera at various scenes of interest, perhaps panning the camera, zooming in or out of particular objects in a scene, etc. Additionally, software according to an optional embodiment of the systems and methods described herein assigns different texture patterns to different colors. For example, red can be converted to stripes on the image and green can be converted to dots, thereby enabling the user to easily differentiate one color from another in the digital image.

Furthermore, the software can display by flashing, highlighting, and/or altering a color or texture pattern, other objects in the image that are identified to map to the same color as the position or region selected by the user. In a further embodiment of this feature, the user can designate one or more specific colors and prompt the software integrated with the cellular phone to configure the phone to flash, highlight, alter the color and/or texture pattern of, or otherwise identify for the user other positions or regions in the image associated with the same color.

As cellular phones are small and convenient to carry, the fact that the software according to the systems and methods described herein can be installed on or otherwise cooperatively operate with the cellular phone enables a color-vision impaired person or other observer to take a digital picture of a scene of interest and ascertain the color of various objects at any time in a unobtrusive manner and without embarrassment.

In one aspect, the invention includes a method of identifying at least one color for a user. The method includes the steps of: allowing the user to capture an image with a camera; displaying the captured image on a display screen; in response to the user selecting a position or region in the displayed image, identifying a set of at least one color parameter associated with the selected position or region; mapping the set of one or more color parameters to one or more or more reference colors; and identifying for the user, and in a form/manner perceptible to the user, the one or more reference colors to which the color parameters of the selected position or region are mapped.

According to one practice, the method includes indicating to the user an additional position or region having corresponding color parameters that map to the same reference colors as the user-selected position or region. According to one embodiment, the additional position or region is indicated by displaying on the screen at least one visual icon, perceptible to the user, identifying the additional position or region as being associated with the reference colors. The displayed visual icon may include one or more of a displayed intensity level, a displayed texture pattern, and a displayed color corresponding to the at least one additional position or region; each of these may be time-varying, for example, flashing or otherwise changing with time.

According to another aspect, the method of identifying at least one color for a user includes allowing the user to capture an image with a camera and to also choose a designated color of interest, for example, a color with respect to which the user is color-vision impaired. The method further includes the steps of displaying the captured image on a display screen; determining an additional position or region in the displayed image having an associated set of one or more color parameters that map to the selected color; and indicating, in a form perceptible to the user, the additional position or region in the displayed image. The method by which the additional position or region is indicated to the user in this aspect is similar to the one described above, for example, by flashing, altering the color and/or texture of, highlighting, etc. the additional position or region.

Embodiments employing other portable devices, such as a personal digital assistant (PDA), a Pocket PC, and a digital camera having a display screen are within the scope of the systems and methods described herein. Further features and advantages of the invention will be apparent from the following description of illustrative embodiments, and from the claims.

### BRIEF DESCRIPTION OF THE DRAWINGS

The following figures depict certain illustrative embodiments of the invention in which like reference numerals refer to like elements. These depicted embodiments are to be understood as illustrative of the invention and not as limiting in any way.
**FIG. 1** depicts a slice through a cube that represents a three-dimensional color space;
**FIG. 2** depicts the color space of **FIG. 1** as seen by a person with red-green color-vision impairment;
**FIGs. 3A-3D** depict cell-phone embodiments of the systems and methods described herein;
**FIGS. 4A-4B** depict position and region selector embodiments, respectively, of the systems and methods described herein;
**FIGs. 5-6** depict alternative embodiments for encoding color information into a format perceptible by a color-vision impaired user;
**FIG. 7** depicts a pseudo-color space comprising a plurality of hatching patterns; and
**FIGs. 8-9** depict various embodiments of the systems and methods described herein, processing an image of the Boston subway map.

### DETAILED DESCRIPTION OF THE ILLUSTRATED EMBODIMENTS

To provide an overall understanding of the invention, certain illustrative practices and embodiments will now be described, including a system and method for identifying one or more colors for a user, in particular, a color-vision impaired observer. The systems and methods described herein can be adapted, modified, and applied to other contexts; such other additions, modifications, and uses will not depart from the scope hereof.

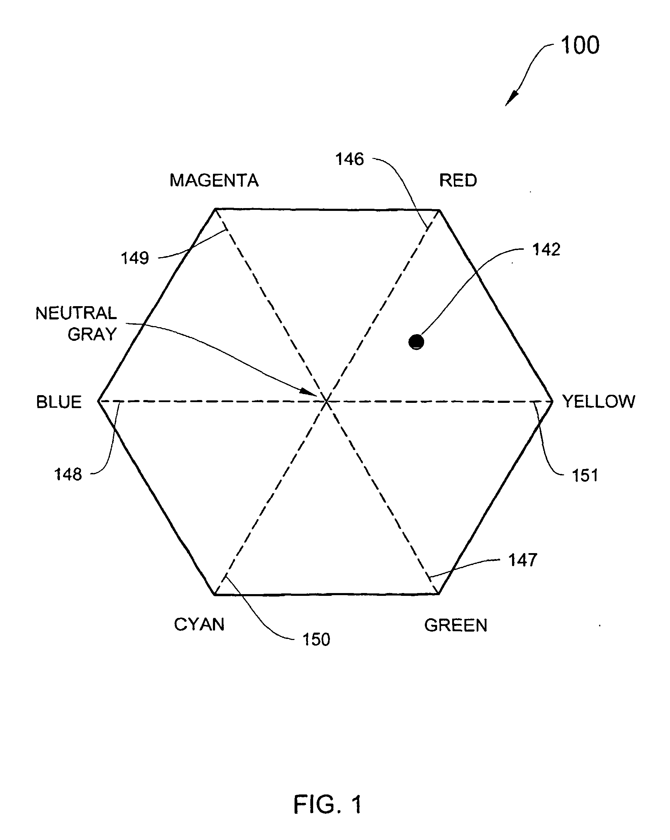

**FIG. 1** depicts a slice **100** through a cube that represents a three-dimensional color space. The color space can be any color space and it will be understood to represents all the possible colors that can be produced by an output device, such as a monitor, color printer, photographic film or printing press, or that appear in an image. The definition of various color spaces are known to those of skill in the art, and the systems and methods described herein may be employed with any of these defined color spaces, with the actual definition selected depending at least in part on the application. These models include the RGB color space model, which uses the three primary colors of transmitted light. The RGB standard is an additive color model as if you add red, green and blue light and you get white. A second known color space model uses reflected light. This subtractive color model attains white by subtracting pigments that reflect cyan, magenta and yellow (CMY) light. Printing processes, the main subtractive users, add black to create the CMYK color space. Aside from RGB and CMYK, there are other alternative color spaces; here are some of the more common:
INDEXED uses 256 colors. By limiting the palette of colors, indexed color can reduce file size while maintaining visual quality.
LAB COLOR (a.k.a. L*a*b and CIELAB) has a lightness component (L) that ranges from 0 to 100, a green to red range from +120 to -120 and a blue to yellow range from +120 to -120. LAB is used by such software as Photoshop as a intermediary step when converting from one color space to another. LAB is based on the discovery that somewhere between the optical nerve and the brain, retinal color stimuli are translated into distinctions between light and dark, red and green, and blue and yellow.
HSL a spherical color space in which L is the axis of lightness, H is the hue (the angle of a vector in a circular hue plan through the sphere), and S is the saturation (purity of the color, represented by the distance from the center along the hue vector).
MULTICHANNEL uses 256 levels of gray in each channel. A single Multi-channel image can contain multiple color modes-e.g., CMYK colors and several spot colors--at the same time.
MONITOR RGB is the color space that reflects the current color profile of a computer monitor.
sRGB is an RGB color space developed by Microsoft and Hewlett-Packard that attempts to create a single, international RGB color space standard for television, print, and digital technologies.
ADOBE RGB contains an extended gamut to make conversion to CMYK more accurate.
YUV (aka Y'CbCr) is the standard for color television and video, where the image is split into luminance (i.e. brightness, represented by Y), and two color difference channels (i.e. blue and red, represented by U and V). The color space for televisions and computer monitors is inherently different and often causes problems with color calibration.
PANTONE is a color matching system maintained by Pantone, Inc.

When discussing color theory in general, particularly as it applies to digital technologies, there are several other important concepts:
HUE-The color reflected from, or transmitted through, an object. In common use, hue refers to the name of the color such as red, orange, or green. Hue is independent of saturation and lightness.
SATURATION (referred to as CHROMINANCE when discussing video)-The strength or purity of a color. Saturation represents the amount of gray in proportion to the hue, measured as a percentage from 0% (gray) to 100% (fully saturated).
LIGHTNESS-Lightness represents the brightness of a color from black to white measured on a scale of 1 to 100.
LOOK-UP TABLE-A look-up table is the mathematical formula or a store of data which controls the adjustment of lightness, saturation hue in a color image or images, and conversion factor for converting between color spaces.

Turning back to **FIG. 1****,** there is depicted a slice **100** through a cube that represents a the R,G, B color space model. This is a representation of the color space known to those of skill in the art. The slice **100** represents a color space in which a plurality of colors can be defined. As shown in **FIG. 1****,** six axes extend from the center point of the slice **100.** Three of these axes are labeled red **146,** green **147** and blue **148** respectively. The other three are labeled magenta **149,** cyan **150** and yellow **151.** Neutral is in the center of the color space. A specific color **142** exists in the color space **100,** and is disposed about midway between the red 146 and yellow axes **151.** This shows the relative amount of each color axis in the specific color **142.** Thus, each point in the slice 100 represents a color that can be defined with reference to the depicted axes.

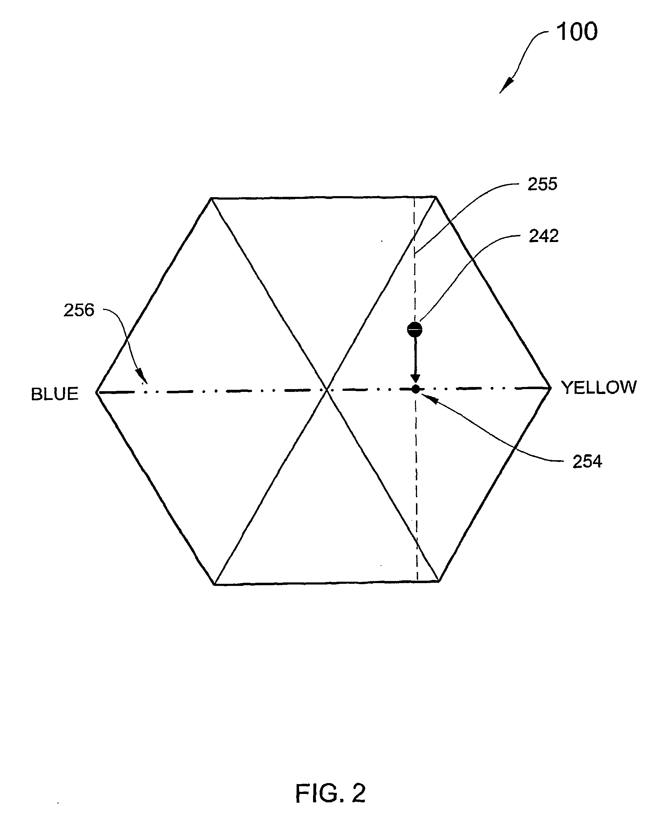

**FIG. 2** depicts the color space **100** as seen by a person with red/green color-vision impairment. As a color vision impaired person having red-green color-vision impairment cannot distinguish red or green, the color space perceived by such a person is compressed or reduced. To such a person, all colors, such as the specific color **242,** are defined only by their position **254** along the blue-yellow axis **256.** Thus, the red component of color **242** is not differentiated by the person and only the component along the blue-yellow axis is differentiated. Thus, this person cannot distinguish between the color **242** and the color **254** that sits on the blue-yellow axis. As such, any information that has been color coded using the color **242** will be indistinguishable from any information that has been color coded using the color **254,** or any other color that falls on line **255.**

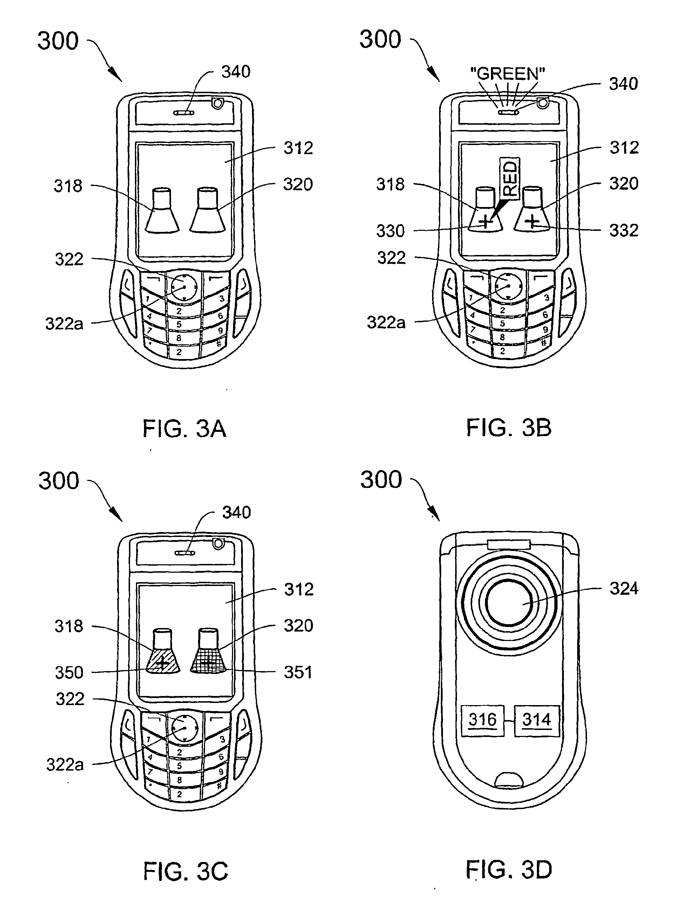

**FIGs. 3A-3D** depict various embodiments of the systems and methods of the invention including a cellular phone **300** equipped with a display screen **312,** a memory/software area **314,** a microprocessor **316,** a position or region selector controller **322,** and a digital camera **324.** **FIGs. 3A-3C** depict a red object **318** and a green object **320.**

Referring to **FIG. 3A**-denoting a cell phone not running the color-identification systems and methods described herein-a red-green color-vision impaired user cannot distinguish between the objects **318** and **320,** which, aside from appearing red and green, respectively, to an ordinary observer, are otherwise essentially identical. The position/region selector controller **322** may include a mouse, a touchpad, a joystick, or other commonly-used pointing or selection devices. A central portion **322a** of the selector controller **322** provides the user an explicit means to actively select, accept, or activate, depending on the context. For example, the user may actively select a desired position for which he or she wants color information.

The systems and methods disclosed herein include, in one embodiment, software stored in memory/software area **314.** The software can be used on images captured with the digital camera **324** resulting in the display of the image on the display screen **312A.** The display screen typically is a liquid crystal display (LCD) screen, but other embodiments including plasma or cathode ray tube (CRT) are within the scope of the disclosure herein.

The camera **324** can generate a file in any format, such as the GIF, JPEG, TIFF, PBM, PGM, PPM, EPSF, X11 bitmap, Utah Raster Toolkit RLE, PDS/VICAR, Sun Rasterfile, BMP, PCX, PNG, IRIS RGB, XPM, Targa, XWD, possibly PostScript, and PM formats on workstations and terminals running the X11 Window System or any suitable image file.

The camera **324** may be used to view a scene in real time. For example, the camera **324** may employ small sensors used in a typical cell phone or consumer digital camera, which can read image data in real time using a scheme called "interline transfer." In this embodiment, charge coupled device (CCD) electronics control exposure rather than a mechanical shutter. The user can then use the cell phone's camera to look at his or her surroundings by panning the cell phone's and looking at the cell phone display to view objects. In this real time setting, too, the systems and methods described herein can be employed by the user to identify colors of objects shown on the display screen.

**FIG. 3B** depicts an embodiment wherein the user moves a position selector **330** (shown in the form of a cross hairpin) over, say, the red object **318.** According to one practice, when the user allows the position selector **330** to sojourn over the red object **318** for at least a predetermined time interval, the systems and methods of the invention produce a floating caption **331** (e.g., a textual bubble or some other visual icon) on the display screen **312,** alerting the user of the color of the selected object **318.** In the figure, the bubble shows the. English name of the color, but other embodiments showing different visual cues perceptible to the user are within the scope of this disclosure.

The cursor can be moved over various parts of the image using the position selector **322** and the cursor will continuously or intermittently display the color of the position in the image that it is over. In an alternative embodiment, the floating caption or other visual icon can appear with an active selection by the user of a particular position or region in the image, without having to pause the cursor over the image.

Alternatively, or additionally, the embodiment of **FIG. 3B** provides auditive cues for the user. For example, when the user points cursor **332** at the green object **320,** an audio sound indicating that the color of the object **320** is green may be emitted from speaker **340.** Alternatively, **340** depicts an ear jack that can broadcast a sound representing the color green to the user. The auditive sound may simply be a voicing uttering the word "green," for example.

**FIG. 3C** depicts yet another cell phone embodiment of the systems and methods described herein. In this embodiment, portions of the image that are red **318** and portions that are green **320** appear with different hatching patterns **350** and **351,** respectively. The user, especially a color-vision impaired user, can then discern red from green, for example, by perceiving the distinct hatching patterns of each color.

**FIG. 3D** depicts a side of the cell phone where the digital camera **324** is found. Microprocessor **316** and memory **314** are also shown in **FIG. 3D****,** with line drawn connecting them to indicate cooperation and data transfer between them.

Although **FIGs. 3A-3D** graphically depict the components of the system **300** as functional block elements, it is understood that these elements can be realized as computer programs or portions of computer programs capable of executing on the microprocessor platform **316** or any data processor platform cooperating with memory unit **314** to thereby configure the data processor as a system according to the invention.

Moreover, although **FIGs. 3A-3D** depict the system **300** as an integrated unit of an imaging system that couples to a data processing system, it is understood that these are only a few embodiments, and that the invention can be embodied as a computer program processing an image file, which includes image data representative of the surface of a membrane. Accordingly, it is not necessary that the camera or imaging device be directly coupled to the data processing system, and instead the images generated by the imaging system can be imported into the data processing system by any suitable technique, including by file transfer over a computer network, or by storing the image file on a disk and mounting and copying the disk into the file system of the data processing. Thus it will be apparent that the camera or imaging system can be remotely situated relative to the data processing system. Thus, the systems and methods described herein can include embodiments wherein users at multiple remote sites create images of interest and deliver the images to a remote processing system that can identify and interpret the colors in the images.

The cellular phone **300** can be a Motorola V300 or any suitable, and preferably commercially-available off-the-shelf cellular phone that is equipped with a digital camera. A Nokia 6630 SmartPhone, having high-resolution and fast imaging and video capability (including zoom and sequence mode and mobile broadband access for multimedia content, live video streaming and video conferencing), MP3 audio output, and sufficient memory, is a particular example of a commercially-available cellular phone suitable for integrating with or implementing the systems and methods described herein.

These cellular phones can be programmed using well-known system development kits such as the Symbian OS (operating system). Additionally, there are companies that offer product design and development services to those seeking professional assistance in creating new software products for use in cellular phones.

In another embodiment, any digital camera device, including digital cameras that do not have cellular phone capability, can be used with this software. The digital camera can be a Canon Powershot S400 or any commercially-available off-the-shelf digital camera. In a further optional embodiment, the camera device may be a web camera of the kind commonly employed to capture image for display on, and transfer over, a computer network. In an additional, optional embodiment, the camera device may be a personal digital assistant (PDA) device that is equipped with a digital camera, including the ViewSonic V36. The systems and methods described herein may also be implemented on, or integrated with, Pocket PCs or other handheld devices.

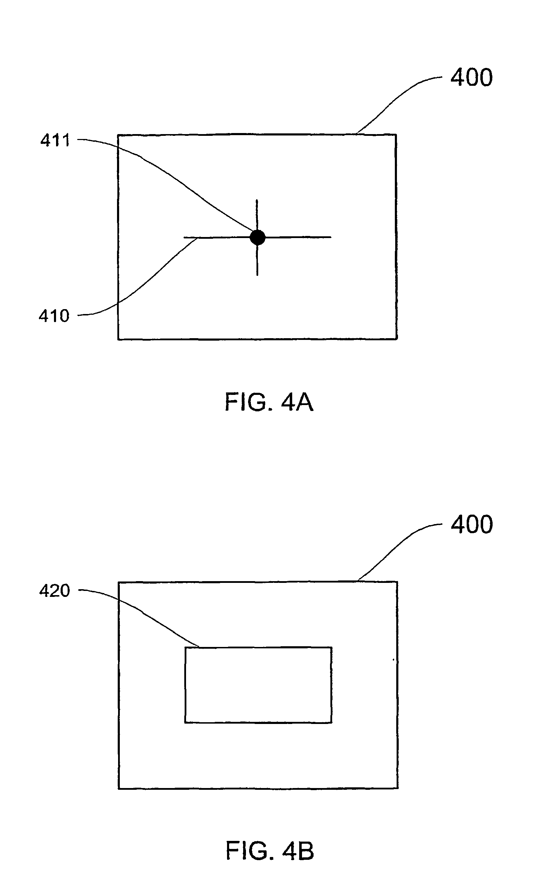

**FIG. 4A** depicts an embodiment wherein the user selects a point (or pixel) on the displayed image **400.** This can be done, for example, by using a cross hairpin configuration **410,** wherein the crossing point **411** is associated with the selected position in the image. An alternative embodiment includes an arrow cursor (not shown) instead of the cross hairpin, wherein the tip of the arrow is associated with the selected position in the image. Other variations do not depart from the scope hereof.

**FIG. 4B** depicts an embodiment wherein the user selects a region **420** of the image **400.** According to one practice, the systems and methods described herein may choose a dominant color present in the region **420** to call out to the user. Alternatively, a discrete number of colors that are found to be present in the region **420** are called out to the user, in a manner similar to those described earlier (i.e., using a floating caption, texture hatching patterns, auditive cues, etc.).

**FIGs. 4A-4B** can also depict embodiments of a viewfinder in a digital camera, where the position **411** and the region **420** are fixed locations, corresponding essentially to a central position or region of the scene of which an image is about to be captured by the camera. In these embodiments, the user can point the camera to an object, superimpose the cross hairpin **411** or the region **420** on the object, and either by pausing over the object or actively prompting the systems and methods described herein (through a click of a selector button, for example), obtain a color "readout" of the object over which the hairpin **411** or the region **420** is superimposed.

In the embodiment where pausing over the object prompts a callout of the color, the digital camera may be enabled with motion estimation software, known in the art of image and video processing, to detect whether there is camera motion. If motion is determined to be below a predetermined threshold (where the threshold is related to the sensitivity of the motion detection algorithm being employed), then the user is assumed to have paused over the object, indicating that he or she wishes to know the color of that object.

Alternatively, or additionally, the camera motion may be determined using techniques known in the electromechanical art of camera motion detection, employing, for example, gyroscopic or other techniques.

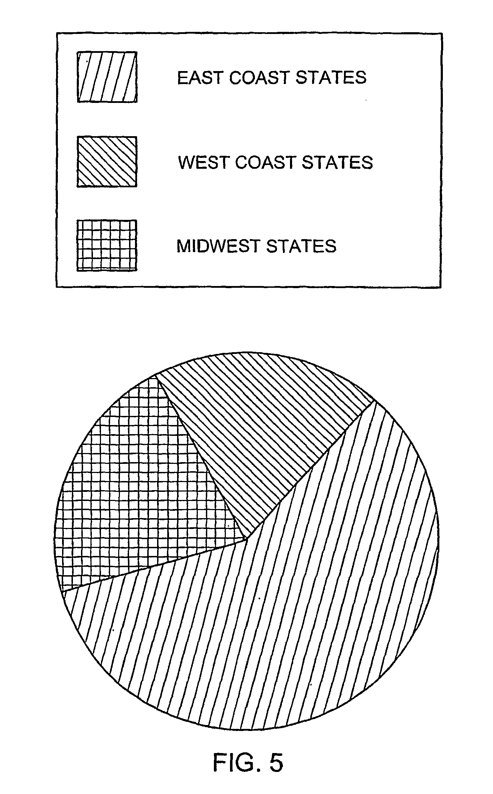

Turning to **FIG. 5**, an alternative embodiment is depicted. Specifically, **FIG. 5** depicts a display wherein in a pie chart is presented to the user. This can be, for example, a scenario where the user holding a cell phone or other camera-enabled handheld device is attending a slide presentation (e.g., a PowerPoint presentation) and wants to discern the various colors present in the projected image.

To the right of the pie chart is a key table that equates different colors on the graph to different kinds of information. In **FIG. 5**, solely for purpose of illustration, the colors are represented by different hatch patterns. In **FIG. 5****,** the key table associates colors (depicted by hatch patterns) with different regions of the country. In this embodiment, the user is capable of rolling the cursor over the different colors presented in the key table. This causes the corresponding portion of the pie chart to alter in a manner that may be detected by a color-vision impaired person. All this can be displayed on the display screen of the handheld device, in real time or by post-processing on a captured and stored image.

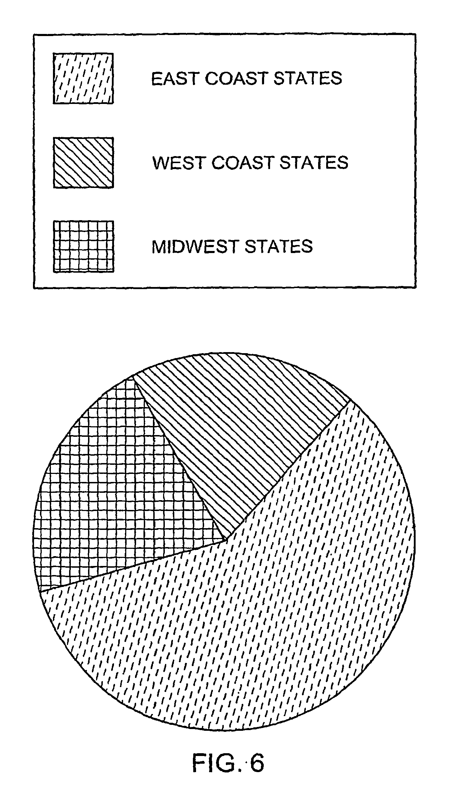

In **FIG. 6****,** the user may place the cursor over the color used in the Key Table to describe "East Coast" sales. By doing this the system knows to flash or otherwise alter those portions of the pie chart that are presented in that color. Alternatively, the user can place the cursor over a portion of the pie chart and the color in the Key Table associated with that color can flash. Optionally, both functions may be simultaneously supported.

Alternatively, when colored data in an image is known to have certain color names, for example, when a map of highway congestion is known to mark congested zones as red and uncongested zones as green, the color-vision impaired person or other user will be able to select a desired color name from an on-screen list of color names, and colors in the image corresponding to that name will flash or be otherwise identified.

Although, **FIG. 5** depicts the image as being redrawn to include a hatch pattern, it shall be understood that shading, grey scale or any other technique may be employed to amend how the selected color information is presented to the user. A black and white bitmap may be created, as well as a grayscale representation that uses for example 256 shades of gray, where each pixel of the grayscale image has a brightness value ranging from 0 (black) to 255 (white).

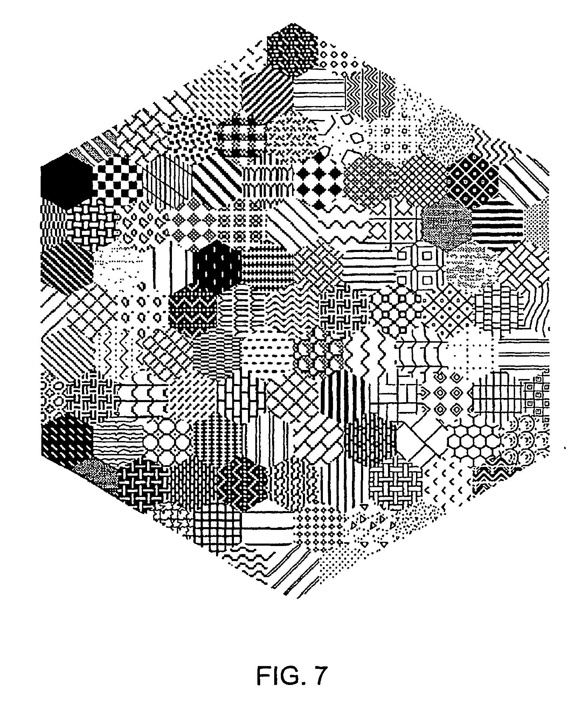

**FIG. 7** depicts a color space that is a pseudo-color space **700** where different colors are represented by different hatching patterns. Color space **700** may act as the intermediate color space described above. In this case, a pixel color value in the original color space called for by the systems and methods described herein can be mapped to a region in color space **700** that has a corresponding hatch pattern. Thus, in this embodiment a selected range of colors from the first color space are mapped to a specific region of this intermediate color space **700.** This selected range of colors are identified as a contiguous area or areas as appropriate in the original image and filled with the respective hatching pattern associated with that selected range of colors. In this way, the output is presented on the display. Thus, the color space **700** may be a perceptual space for the user, and colors may be mapped to this perceptual space.

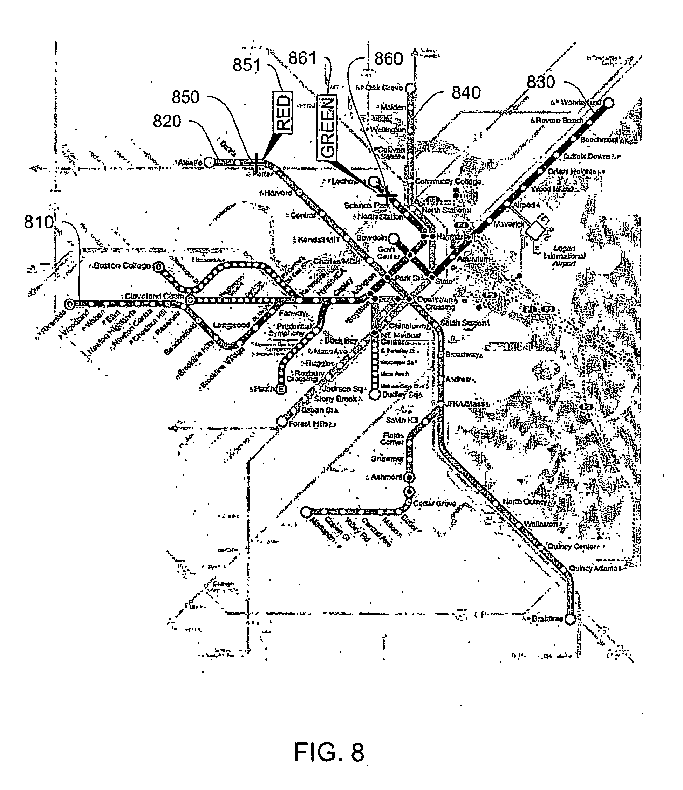

**FIG. 8** depicts a Boston subway map **800** including color-coded subway lines. For example, the Green Line **810,** the Red Line **820,** the Blue Line **830,** and the Orange Line **840** are labeled in the figure. A color-vision impaired observer standing in a subway station, looking at the map **800,** is likely to encounter problems trying to discern, for example, the Green Line **810** from the Red Line **820.** However, using a cell phone or a digital camera enabled with the systems and methods described herein, the observer can capture an image of the map from, say, a wall where the map is mounted on at the station. As the Red and Green Lines look alike to the observer, the observer can select at least two positions, one position **850** on the Red Line **820** and the other position **860** on the Green Line **810,** to identify their respective colors. The observer can select more than these two positions or regions on the image; however, for the purpose of illustration, two positions will suffice. The systems and methods according to one embodiment of the invention and executing on the cell phone or the digital camera or other handheld device operated by the observer produce, a floating text caption **851** indicating the color "RED" to the observer and another caption **861** indicating the color "GREEN." In this manner, the observer is able to discern the colors of the various subway lines.

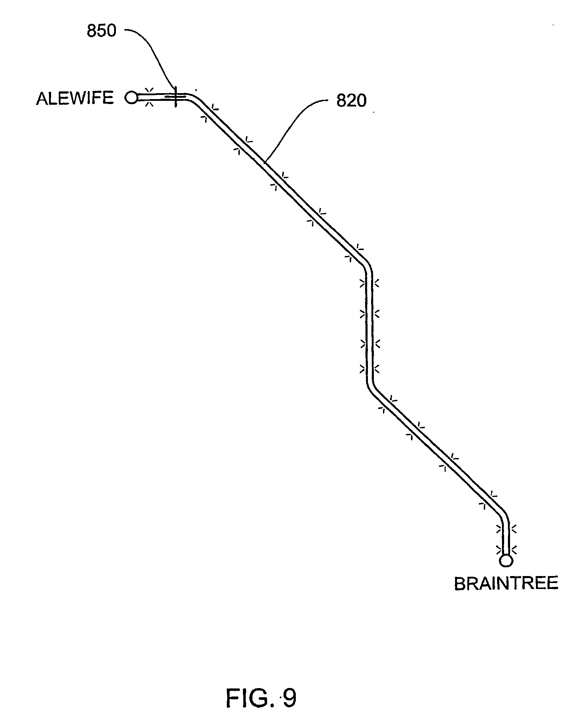

**FIG. 9** depicts an embodiment of the systems and methods described herein wherein the user selects a position **850** on the Red Line **810** of **FIG. 8****,** and wishes to see all other positions or regions in the image corresponding to the same color as that of the location **850.** According to one practice, and in response to the user selecting the color to be identified, the systems and methods described herein determine at least one position or region in the displayed image that correspond to the same color, and convey, in a form perceptible to the user, the information by, for example, highlighting the Red Line (assuming only the Red Line appears as a variation of the color red in the map); time-varying the intensity of the positions corresponding to the Red Line, changing the color of the Red Line to one that the user can perceive and distinguish over the other colors, introduce a unique hatching pattern for the Red Line, or a combination of these and other techniques for conveying color information to the user. In an embodiment wherein regions having colors mapping to the same identified color associated with the selected region or position are sought, the systems and methods described herein can employ image segmentation systems and methods known in the arts of image and video processing, pattern recognition, and artificial intelligence, to segment the image into various objects, and search for colors within the segmented image.

In one aspect, the systems and methods described herein discretize a continuous, or practically continuous, range of colors that can appear in an image, into a set of reference colors. For example, various shades of red are mapped to "Red." In one embodiment, when the user selects a position having any of those shades that map to "Red," the floating bubble would indicate "Red." Similarly, when the user is interested in the some or all positions or regions in the image having the color red, the systems and methods described herein map any of the shades of red (or whatever other range of colors is determined a priori to map to "Red") are highlighted or otherwise exposed to the user in a form perceptible to the user.

This is essentially a form of "quantization" of the color space, associating with each continuous pocket of the color space one color representative of that pocket. Alternatively, referring to **FIG. 1**, the cube representing the color space can be divided into mutually exclusive, collectively exhaustive subsets, with each subset having one color representative of all colors present in that respective subset.

As discussed above, the imaging system can be realized as a software component operating on a cell phone or other device having an image capture device and a data processing system. In that embodiment, the imaging software can be implemented as a C language computer program, or a computer program written in any high level language including C++, Fortran, Java or Basic. Additionally, in an embodiment where microcontrollers or DSPs are employed, the imaging software can be realized as a computer program written in microcode or written in a high level language and compiled down to microcode that can be executed on the platform employed. The development of such image processing systems is known to those of skill in the art. Additionally, general techniques for high level programming are known, and set forth in, for example, Stephen G. Kochan, Programming in C, Hayden Publishing (1983).

Many equivalents to the specific embodiments of the invention and the specific methods and practices associated with the systems and methods described herein exist. For example, the systems and methods described herein can work with video images of the type captured by digital camcorders and video devices and are not limited to still images. Accordingly, the invention is not to be limited to the embodiments, methods, and practices disclosed herein, but is to be understood from the following claims, which are to be interpreted as broadly as allowed under the law.

## Claims

1. A method of identifying at least one color for a user, comprising:
allowing the user to capture an image with a camera (324);
displaying the image on a display screen (312);
in response to the user selecting a position in the displayed image (400), identifying a set of at least one color parameter associated with the selected position;
mapping the set of at least one color parameter to a selected subset of a plurality of reference colors; and
identifying the selected subset of the reference colors for the user.

2. The method of claim 1, including indicating to the user at least one additional position in the displayed image (400) having an associated additional set of at least one color parameter, wherein the additional set of at least one color parameter maps to the selected subset of the plurality of reference colors, and optionally a) wherein indicating the at least one additional position (860) includes displaying on the screen (312) at least one visual icon (861) identifying the at least one additional position as being associated with the selected subset of the reference colors, and optionally wherein the at least one visual icon (861) includes at least one of a textual icon and a graphical icon, or optionally b) wherein indicating the at least one additional position includes changing, in a form perceptible to the user, at least one of a displayed intensity level, a displayed texture pattern, and a displayed color corresponding to the at least one additional position, and optionally wherein a time rate of change of the displayed intensity level is employed to indicate a feature of the associated additional set of at least one parameter.

3. The method of claim 1, including the user at least partially controlling the camera (324), and optionally wherein the at least partially controlling includes at least one of aiming the camera (324) at a target scene of interest, adjusting a focal length of the camera, adjusting an image magnification feature of the camera, panning the camera, adjusting an aperture diameter of the camera, adjusting a light-sensitivity of the camera, and adjusting a shutter speed of the camera.

4. The method of claim 1, wherein the identifying includes conveying to the user, in a form perceptible to the user, information representative of the selected subset of the reference colors, and optionally wherein the conveying includes providing the user with at least one of a visual indicator and an auditive indicator, and optionally a) wherein providing the visual indicator includes displaying on the screen (312) at least one visual icon identifying the selected subset of the reference colors, and optionally wherein a subset of the at least one visual icon includes at least one of a textual icon (851) and a graphical icon, or optionally b) wherein providing the visual indicator includes changing, in a form perceptible to the user, at least one of a displayed intensity level, a displayed texture pattern, and a displayed color associated with the selected position in the displayed image, and optionally wherein a time rate of change of the displayed intensity level is employed to indicate a feature of the associated set of at least one color parameter, or optionally c) wherein providing the auditive indicator includes playing for the user and on a speaker at least one name identifying the selected subset of the reference colors.

5. The method of claim 1, including the camera communicating with a handheld electronic device (300) housing the display screen (312), the handheld device at least partially controlled by the user, and optionally wherein the handheld device includes at least one of a mobile telephone, a personal digital assistant, a Pocket PC, and a digital camera having a display screen.

6. The method of claim 1, wherein the image capture device is integrated with the display screen.

7. The method of claim 1, including
in response to the user selecting an additional position in the displayed image, identifying an additional set of at least one color parameter associated with the selected additional position;
mapping the additional set of at least one color parameter to an additional subset of a plurality of reference colors; and
identifying the additional subset of the selected reference colors for the user.

8. A system for identifying at least one color for a user, comprising:
a handheld device (300) having a data processor (316) and memory (314) configured to execute at least one software application on the handheld device:
an image capture device (324) in communication with the handheld device (300) and configured to provide image date to the handheld device;
a display screen (312) integrated with the image capture device (324) and the handheld device (300) for displaying the image data,
wherein the user at least partially controls the image capture device (324) to acquire the image data and allows a subset of the at least one software application to determine color information associated with a selected position, map the color information to a selected subset of a plurality of reference colors, and identify the selected subset of reference colors for the user, and **characterized by** a position selector (322) at least partially controlled by the user to select a position in the image data.

## Patentansprüche

1. Verfahren zum Identifizieren wenigstens einer Farbe für einen Benutzer, folgendes umfassend:
Ermöglichen, dass der Benutzer mit einer Kamera (324) ein Bild erfassen kann;
Darstellen des Bildes auf einem Bildschirm (312);
in Reaktion darauf, dass der Benutzer im dargestellten Bild (400) eine Position auswählt, Identifizieren eines Satzes von wenigstens einem Farbparameter, der zu der ausgewählten Position gehört;
Zuordnen des Satzes von wenigstens einem Farbparameter zu einer ausgewählten Teilmenge aus mehreren Referenzfarben; und
Identifizieren der ausgewählten Teilmenge der Referenzfarben für den Benutzer.

2. Verfahren nach Anspruch 1, folgendes umfassend: Anzeigen wenigstens einer zusätzlichen Position im dargestellten Bild (400) für den Benutzer, die einen zugehörigen zusätzlichen Satz von wenigstens einem Farbparameter hat, wobei der zusätzliche Satz von wenigstens einem Farbparameter der ausgewählten Teilmenge aus den mehreren Referenzfarben zugeordnet ist, und wobei optional a) das Anzeigen der wenigstens einen zusätzlichen Position (860) die Darstellung wenigstens eines sichtbaren Icons (861) auf dem Bildschirm (312) umfasst, das die wenigstens eine zusätzliche Position als zur ausgewählten Teilmenge der Referenzfarben gehörend identifiziert, und wobei optional das wenigstens eine sichtbare Icon (861) wenigstens eines von einem Texticon und einem grafischen Icon umfasst, oder wobei optional b) das Anzeigen der wenigstens einen zusätzlichen Position das Verändern in einer für den Benutzer wahrnehmbaren Form von wenigstens einem der folgenden Punkte umfasst: einem dargestellten Intensitätsniveau, einem dargestellten Texturmuster und einer dargestellten Farbe, entsprechend der wenigstens einen zusätzlichen Position, und wobei optional eine zeitliche Veränderungsrate des dargestellten Intensitätsniveaus verwendet wird, um eine Eigenschaft des zugehörigen zusätzlichen Satzes von wenigstens einem Parameter anzuzeigen.

3. Verfahren nach Anspruch 1, das die wenigstens teilweise Steuerung der Kamera (324) durch den Benutzer umfasst, und wobei optional das wenigstens teilweise Steuern wenigstens einen der folgenden Punkte umfasst: Ausrichten der Kamera (324) auf eine interessierende Zielszene, Einstellen der Brennweite der Kamera, Einstellen einer Bildvergrößerungsfunktion der Kamera, Schwenken der Kamera, Einstellen eines Blendendurchmessers der Kamera, Einstellen einer Lichtempfindlichkeit der Kamera und Einstellen einer Shuttergeschwindigkeit der Kamera.

4. Verfahren nach Anspruch 1, wobei das Identifizieren folgendes umfasst: Übermitteln an den Benutzer, in einer für den Benutzer wahrnehmbaren Form, von Information, die die ausgewählte Teilmenge der Referenzfarben repräsentiert, und wobei optional das Übermitteln folgendes umfasst: Bereitstellen für den Benutzer von wenigstens einem von einer sichtbaren Kennung und einer auditiven Kennung, und wobei optional a) das Bereitstellen der sichtbaren Kennung die Darstellung wenigstens eines sichtbaren Icons auf dem Bildschirm (312) umfasst, das die ausgewählte Teilmenge der Referenzfarben identifiziert, und wobei optional eine Teilmenge des wenigstens einen sichtbaren Icons wenigstens eines von einem Texticon (851) und einem grafischen Icon umfasst, oder wobei optional b) das Bereitstellen der sichtbaren Kennung das Verändern in einer für den Benutzer wahrnehmbaren Form von wenigstens einem der folgenden Punkte umfasst: einem dargestellten Intensitätsniveau, einem dargestellten Texturmuster und einer dargestellten Farbe, die der ausgewählten Position im dargestellten Bild zugeordnet sind, und wobei optional eine zeitliche Veränderungsrate des dargestellten Intensitätsniveaus verwendet wird, um eine Eigenschaft des zugehörigen Satzes von wenigstens einem Farbparameter anzuzeigen, oder wobei optional c) das Bereitstellen der auditiven Kennung das Abspielen für den Benutzer und auf einem Lautsprecher von wenigstens einem Namen umfasst, der die ausgewählte Teilmenge der Referenzfarben identifiziert.

5. Verfahren nach Anspruch 1, wobei es umfasst, dass die Kamera in Verbindung mit einem tragbaren elektronischen Gerät (300) steht, das den Bildschirm (312) aufnimmt, wobei das tragbare Gerät wenigstens teilweise durch den Benutzer gesteuert wird und wobei optional das tragbare Gerät wenigstens eines der folgenden umfasst: ein Mobiltelefon, einen persönlicher digitaler Assistent (PDA), einen Pocket-PC und eine Digitalkamera mit einem Bildschirm.

6. Verfahren nach Anspruch 1, wobei das Bilderfassungsgerät mit dem Bildschirm zusammengefasst ist.

7. Verfahren nach Anspruch 1, folgendes umfassend:
in Reaktion darauf, dass der Benutzer eine zusätzliche Position im dargestellten Bild auswählt, Identifizieren eines zusätzlichen Satzes von wenigstens einem Farbparameter, der zu der ausgewählten zusätzlichen Position gehört;
Zuordnen des zusätzlichen Satzes von wenigstens einem Farbparameter zu einer zusätzlichen Teilmenge aus mehreren Referenzfarben; und
Identifizieren der zusätzlichen Teilmenge der ausgewählten Referenzfarben für den Benutzer.

8. System zur Identifikation wenigstens einer Farbe für einen Benutzer, folgendes umfassend:
ein tragbares Gerät (300) mit einem Datenprozessor (316) und Speicher (314), die dafür eingerichtet sind, wenigstens eine Software-Anwendung auf dem tragbaren Gerät auszuführen;
ein Bilderfassungsgerät (324), das mit dem tragbaren Gerät (300) verbunden ist und das dafür eingerichtet ist, dem tragbaren Gerät Bilddaten zur Verfügung zu stellen;
ein Bildschirm (312), der mit dem Bilderfassungsgerät (324) und dem tragbaren Gerät (300) integriert ausgeführt ist, zur Darstellung der Bilddaten,
wobei der Benutzer das Bilderfassungsgerät (324) wenigstens teilweise steuert, um Bilddaten zu erfassen, und er es einer Teilmenge der wenigstens einen Software-Anwendung gestattet, Farbinformation zu bestimmen, die mit einer ausgewählten Position verbunden ist, die Farbinformation einer ausgewählten Teilmenge aus mehreren Referenzfarben zuzuordnen und die ausgewählte Teilmenge von Referenzfarben für den Benutzer zu identifizieren,
und **gekennzeichnet durch** eine Positionsauswahlvorrichtung (322), die wenigstens teilweise **durch** den Benutzer gesteuert wird, um eine Position in den Bilddaten auszuwählen.

## Revendications

1. Procédé d'identification d'au moins une couleur pour un utilisateur, comprenant :
la possibilité pour l'utilisateur de capturer une image avec un appareil photographique (324) ;
l'affichage de l'image sur un écran d'affichage (312) ;
en réponse à l'utilisateur sélectionnant une position dans l'image affichée (400), l'identification d'un ensemble d'au moins un paramètre de couleur associé avec la position sélectionnée ;
la représentation mappage de l'ensemble d'au moins un paramètre de couleur sur un sous-ensemble sélectionné d'une pluralité de couleurs de référence ; et
l'identification du sous-ensemble sélectionné des couleurs de référence pour l'utilisateur.

2. Procédé selon la revendication 1, incluant l'indication à l'utilisateur d'au moins une position additionnelle dans l'image affichée (400) ayant un ensemble additionnel associé d'au moins un paramètre de couleur, dans lequel l'ensemble additionnel d'au moins un paramètre de couleur se représente sur le sous-ensemble sélectionné de la pluralité de couleurs de référence, et facultativement a) dans lequel l'indication de l'au moins une position additionnelle (860) inclut l'affichage sur l'écran (312) d'au moins une icône visuelle (861) identifiant l'au moins une position additionnelle comme étant associée avec le sous-ensemble sélectionné des couleurs de référence, et facultativement dans lequel l'au moins une icône visuelle (861) inclut au moins l'une d'une icône textuelle et d'une icône graphique, ou facultativement b) dans lequel l'indication de l'au moins une position additionnelle inclut le changement, sous une forme perceptible par l'utilisateur, d'au moins l'un d'un niveau d'intensité affiché, d'un motif de texture affiché, et d'une couleur affichée correspondant à l'au moins une position additionnelle, et facultativement dans lequel un taux temporel de changement du niveau d'intensité affiché est employé pour indiquer une caractéristique de l'ensemble additionnel associé d'au moins un paramètre.

3. Procédé selon la revendication 1, incluant l'utilisateur commandant au moins partiellement l'appareil photographique (324), et facultativement dans lequel la commande au moins partielle inclut au moins l'un de la visée de l'appareil photographique (324) sur une scène cible d'intérêt, de l'ajustement d'une longueur focale de l'appareil photographique, de l'ajustement d'une caractéristique d'agrandissement d'image de l'appareil photographique, d'un panoramique de l'appareil photographique, de l'ajustement d'un diamètre d'ouverture de l'appareil photographique, de l'ajustement d'une sensibilité à la lumière de l'appareil photographique, et de l'ajustement d'une vitesse d'obturateur de l'appareil photographique.

4. Procédé selon la revendication 1, dans lequel l'identification inclut de transporter jusqu'à l'utilisateur, sous une forme perceptible par l'utilisateur, des informations représentatives du sous-ensemble sélectionné des couleurs de référence, et facultativement dans lequel le transport inclut de fournir à l'utilisateur au moins l'un d'un indicateur visuel et d'un indicateur sonore, et facultativement a) dans lequel la fourniture de l'indicateur visuel inclut l'affichage sur l'écran (312) d'au moins une icône visuelle identifiant le sous-ensemble sélectionné des couleurs de référence, et facultativement dans lequel un sous-ensemble de l'au moins une icône visuelle inclut au moins l'une d'une icône textuelle (851) et d'une icône graphique, ou facultativement b) dans lequel la fourniture de l'indicateur visuel inclut le changement, sous une forme perceptible par l'utilisateur, d'au moins l'un d'un niveau d'intensité affiché, d'un motif de texture affiché, et d'une couleur affichée associée avec la position sélectionnée dans l'image affichée, et facultativement dans lequel un taux temporel de changement du niveau d'intensité affiché est employé pour indiquer une caractéristique de l'ensemble associé d'au moins un paramètre de couleur, ou facultativement c) dans lequel la fourniture de l'indicateur sonore inclut de jouer pour l'utilisateur et sur un haut-parleur au moins un nom identifiant le sous-ensemble sélectionné des couleurs de référence.

5. Procédé selon la revendication 1, incluant l'appareil photographique communicant avec un dispositif électronique portable (300) hébergeant l'écran d'affichage (312), le dispositif portable au moins partiellement commandé par l'utilisateur, et facultativement dans lequel le dispositif portable inclut au moins l'un d'un téléphone mobile, d'un assistant numérique personnel, d'un PC de poche, et d'un appareil photographique numérique ayant un écran d'affichage.

6. Procédé selon la revendication 1, dans lequel le dispositif de capture d'image est intégré avec l'écran d'affichage.

7. Procédé selon la revendication 1, incluant
en réponse à l'utilisateur sélectionnant une position additionnelle dans l'image affichée,
l'identification d'un ensemble additionnel d'au moins un paramètre de couleur associé avec la position additionnelle sélectionnée ;
la représentation de l'ensemble additionnel d'au moins un paramètre de couleur sur un sous-ensemble additionnel d'une pluralité de couleurs de référence ; et
l'identification du sous-ensemble additionnel des couleurs de référence sélectionnées pour l'utilisateur.

8. Système pour identifier au moins une couleur pour un utilisateur, comprenant :
un dispositif portable (300) ayant un processeur de données (316) et une mémoire (314) configurés pour exécuter au moins une application logicielle sur le dispositif portable ;
un dispositif de capture d'image (324) en communication avec le dispositif portable (300) et configuré pour fournir des données d'image au dispositif portable ;
un écran d'affichage (312) intégré avec le dispositif de capture d'image (324) et le dispositif portable (300) pour afficher les données d'image,
dans lequel l'utilisateur commande au moins partiellement le dispositif de capture d'image (324) pour acquérir les données d'image et permet à un sous-ensemble de l'au moins une application logicielle de déterminer des informations de couleur associées avec une position sélectionnée, de représenter les informations de couleur sur un sous-ensemble sélectionné d'une pluralité de couleurs de référence, et d'identifier le sous-ensemble sélectionné de couleurs de référence pour l'utilisateur, et
**caractérisé par**
un sélecteur de position (322) au moins partiellement commandé par l'utilisateur pour sélectionner une position dans les données d'image.
